Europäisches Patentamt

European Patent Office

Office européen des brevets

(19)

(11) Veröffentlichungsnummer: **0 503 294 A2**

# EUROPÄISCHE PATENTANMELDUNG

(12)

(21) Anmeldenummer: **92102330.5**

(22) Anmeldetag: **12.02.92**

(51) Int. Cl.5: **C08G 65/22**, C07C 51/00,
C07C 51/58

(30) Priorität: **14.02.91 DE 4104465**

(43) Veröffentlichungstag der Anmeldung:
**16.09.92 Patentblatt 92/38**

(84) Benannte Vertragsstaaten:
**DE FR GB IT NL**

(71) Anmelder: **HOECHST AKTIENGESELLSCHAFT
Postfach 80 03 20
W-6230 Frankfurt am Main 80(DE)**

(72) Erfinder: **Meyer, Matthias, Dr.
Platanenstrasse 12
W-6230 Frankfurt am Main 80(DE)**

(54) Verfahren zur Herstellung von Perfluorpolyetheracylfluoriden.

(57) Die Erfindung betrifft ein Verfahren zur Herstellung von Perfluorpolyetheracylfluoriden der Formel (I)

$$F_3C(CF_2)_2\text{-}O\text{-}[CF(CF_3)CF_2\text{-}O\text{-}]_nCF(CF_3)COF \qquad (I)$$

wobei n eine ganze Zahl von 0 - 60 bedeutet, bei dem man Hexafluorpropylenoxid in Gegenwart von CsF und einem Polyethylenglykoldimethylether oligomerisiert und das dabei entstehende Reaktionsgemisch mit mindestens einer der in einem aprotischen Lösungsmittel gelösten Halogenverbindungen HCl, HBr, $BCl_3$, $PCl_3$, $SCl_2$ und $R_mSiCl_{4\text{-}m}$, wobei m = 0, 1, 2 oder 3 und R = $C_1$-$C_3$-Alkyl oder Phenyl ist, umsetzt.

EP 0 503 294 A2

Rank Xerox (UK) Business Services

Die Erfindung betrifft ein Verfahren zur Herstellung von Perfluorpolyetheracylfluoriden der Formel (I)

$$F_3C(CF_2)_2\text{-}O\text{-}[CF(CF_3)CF_2\text{-}O\text{-}]_nCF(CF_3)COF \qquad (I)$$

wobei n eine ganze Zahl von 0 - 60 bedeutet, aus dem Roholigomer von Hexafluorpropylenoxid (HFPO).

In EP-A-0 154 297 ist die Oligomerisation von HFPO in Gegenwart von CsF und Polyethylenglykoldimethylethern, insbesondere Tetraethylenglykoldimethylether beschrieben. Das HFPO-Roholigomer fällt dabei im allgemeinen als Gemisch mit einer Molmassenverteilung von ca. 300 - 20 000 g/mol an; es enthält jedoch noch CsF und beträchtliche Mengen der genannten Lösungsmittel, die bei der anschließenden Umsetzung des HFPO-Oligomers mit elementarem Fluor zu perfluorierten Polyethern ("Endgruppenstabilisierung") Nebenprodukte bilden, die die perfluorierten Ether verunreinigen.

In EP-A-0 154 297, Seite 18, wird ein Verfahren zur Abtrennung von Tetraethylenglykoldimethylether aus dem HFPO-Roholigomer beschrieben. Nach dessen Verseifung zu Perfluorpolyethercarbonsäuren werden diese durch mehrmalige Estraktion mit Diethylether vom Tetraethylenglykoldimethylether befreit. Der Restgehalt an Diethylether wird durch Erhitzen unter vermindertem Druck von den Perfluorpolyethercarbonsäuren abgetrennt. Diese werden anschließend mit elementarem Fluor zu Perfluorpolyethern "endgruppenstabilisiert".

Es wurde nun gefunden, daß man das HFPO-Roholigomer in reines Perfluorpolyetheracylfluorid, frei von den genannten Lösungsmitteln, umwandeln kann. Dieses kann dann direkt mit $F_2$ in Perfluorpolyether überführt werden. Dadurch entfällt die umständliche Verseifung des Roholigomers zu Perfluorpolyethercarbonsäuren und deren anschließende Trocknung.

Gegenstand der Erfindung ist ein Verfahren zur Herstellung von Perfluorpolyetheracylfluoriden der Formel (I)

$$F_3C(CF_2)_2\text{-}O\text{-}[CF(CF_3)CF_2\text{-}O\text{-}]_nCF(CF_3)COF \qquad (I)$$

wobei n eine ganze Zahl von 0-60 bedeutet, dadurch gekennzeichnet, daß man Hexafluorpropylenoxid in Gegenwartvon CsF und einem Polyethylenglykoldimethylether oligomerisiert und das dabei entstehende Reaktionsgemisch mit mindestens einer der in einem aprotischen Lösungsmittel gelösten Halogenverbindungen HCl, HBr, $BCl_3$, $PCl_3$, $SCl_2$ und $R_mSiCl_{4\text{-}m}$, wobei m = 0, 1, 2 oder 3 und R = $C_1$-$C_3$-Alkyl oder Phenyl ist, umsetzt.

Im allgemeinen verwendet man 1 bis 3 Mol der Halogenverbindung pro Mol CsF in dem bei der Oligomerisation entstehenden Reaktionsgemisch (Roholigomer).

Bevorzugte Halogenverbindungen sind HCl und $ClSi(CH_3)_3$ (Chlortrimethylsilan).

Das eingesetzte aprotische Lösungsmittel kann polar oder apolar sein; vorzugsweise verwendet man Diethylether, Tetrahydrofuran, tert.-Butylmethylether, Aceton, Acetonitril, Chloroform, Methylenchlorid, Pentan, Hexan, Cyclohexan oder Heptan, insbesondere Cyclohexan oder Hexan.

Die Menge an Lösungsmittel beträgt im allgemeinen 0.5 bis 3, vorzugsweise 0.8 bis 1.3 Volumenteile pro Volumenteil Roholigomer.

Die Reaktionstemperatur beträgt im allgemeinen -40 °C bis + 80 °C; vorzugsweise arbeitet man bei Raumtemperatur oder in deren Nähe.

Beim erfindungsgemäßen Verfahren kann das gemäß EP-A-0154297 oder GB-PS 1033574 erhaltene HFPO-Roholigomer ohne Vorreinigung eingesetzt werden. Dazu wird die Emulsion aus dem Oligomer, CsF und dem eingesetzten Polyethylenglykoldimethylether mit der Lösung der Halogenverbindung in dem aprotischen Lösungsmittel versetzt und gerührt. Die Umsetzung ist im wesentlichen beendet, wenn die Trübung des Gemisches durch ausfallendes Cs-Salz nicht mehr zunimmt. Im allgemeinen ist die Mindestdauer etwa 30 Minuten. Eine genauere Kontrolle des Umsetzungsgrades ist durch $^{19}$F-NMR-Analyse von Proben während der Umsetzung möglich.

Nach Filtration enthält die untere Phase außer dem gewünschten Acylfluorid (I) noch etwa 5000 - 10 000 ppm des eingesetzten Lösungsmittels, das beispielsweise durch Dünnschichtdestillation abgetrennt wird. Die obere Phase enthält die Hauptmenge des eingesetzten Lösungsmittels und den bei der Oligomerisation des HFPO eingesetzten Polyethylenglykoldimethylether.

Die erhaltenen Produkte (I) fallen als wasserklare, farblose und je nach Molmasse mehr oder weniger viskose, hydrolyseempfindliche Flüssigkeiten an, die durch Umsetzung mit elementarem Fluor in die "endgruppenstabilisierten" Perfluorether $F_3C(CF_2)_2\text{-}O\text{-}[CF(CF_3)CF_2\text{-}O\text{-}]_nCF_2\text{-}CF_3$ überführt werden können.

Den Polymerisationsgrad n der Perfluorpolyetheracylfluoride kann man durch die Temperatur bei der Oligomerisation des HFPO steuern; je tiefer die Temperatur gewählt wird, umso höher ist der Wertvon n. Besonders interessant sind Werte von n = 10 bis n = 60. Es entsteht aber stets ein Gemisch von HFPO-Oligomeren verschiedener Kettenlänge und daher ein Gemisch von entsprechenden Acylfluoriden (I).

Die aus den Acylfluoriden hergestellten Perfluorpolyether werden beispielsweise in der Elektronik- und der chemischen Industrie als Inertflüssigkeiten und Schmiermittel eingesetzt.

Beispiele

Versuchsbericht (Herstellung des HFPO-Oligomeren)

Eine Lösung von 20 g CsF in 50 ml Tetraethylenglykoldimethylether (Tetraglyme) und 56 g Hexafluorpropylenoxid wurden unter Stickstoffatmosphäre in einem 4 l V-4-A Autoklaven vorgelegt. Zusätzlich wurden 250 ml ®Frigen F 113 ($CCl_2F$-$CF_2Cl$) zur Verdünnung hinzugefügt. Bei einer Temperatur von -40°C bis 0°C wurden dann 4000 g Hexafluorpropylenoxid über einen Zeitraum von 8 Stunden unter guter Durchmischung eingeleitet. Nach Reaktionsende wurde auf Raumtemperatur erwärmt und anschließend das HFPO-Roholigomer unter Stickstoffatmosphäre abgelassen. Dieses Roholigomer wurde in den folgenden Beispielen eingesetzt.

Beispiel 1

Es wurden 200 g des mit F113, CsF und Tetraglyme verunreinigten HFPO-Roholigomers in einen 250 ml-Rührkolben gegeben. Das Roholigomer mit einem mittleren Molekulargewicht von 9700 g/mol wurde mit einer Lösung von 2 g Chlortrimethylsilan in 100 ml Diethylether bei Raumtemperatur unter Stickstoffatmosphäre 30 Minuten lang gerührt. Nach Phasentrennung wurde die das Produkt (I) enthaltende untere Phase abgelassen, vom Cäsiumchlorid filtriert und F113 sowie der (geringe) Gehalt an Diethylether im Vakuum ($10^{-2}$ mbar) bei 100°C abgezogen. 183 g Perfluorpolyetheracylfluorid (I) (98.9 % Ausbeute) wurden gewonnen. Mit $^1$H-NMR-Restprotonenanalyse wurden noch 0.008 Gew.-% Diethylether und 0.002 Gew.-% Tetraglyme im Produkt (I) nachgewiesen. Der Reinheitsgrad von (I) betrug 99.99 %.

Beispiel 2

Es wurde wie im Beispiel 1 gearbeitet, jedoch mit 100 ml Tetrahydrofuran statt Diethylether.
Mit $^1$H-NMR-Restprotonenanalyse konnten noch 0.1 mg THF und 0.04 mg Tetraglyme pro g Produkt (I) nachgewiesen werden. Die Ausbeute betrug 184.3 g, d.h. 99.6 %.

Beispiel 3

Es wurde wie in Beispiel 1 gearbeitet, jedoch mit 100 ml n-Hexan statt Diethylether. Mit der NMR-Analyse wurden noch etwa 0.05 mg Tetraglyme pro g Produkt (I) nachgewiesen. Die Ausbeute betrug 182.8 g, d.h. 98.8 %.

Beispiel 4

Es wurde wie in Beispiel 1 gearbeitet, jedoch mit 100 ml tert.-Butylmethylether statt Diethylether. Mit der NMR-Analyse wurden noch etwa 0.54 mg tert.-Butylmethylether und 0.14 mg Tetraglyme pro g Produkt (I) nachgewiesen. Die Ausbeute betrug 181.9 g, d.h. 98.3 %.

Beispiel 5

Es wurde gearbeitet wie in Beispiel 1, jedoch mit einer Lösung von 0.68 g gasförmiger HCl in 100 ml n-Hexan (statt einer Lösung von Chlortrimethylsilan in Diethylether). Mit der NMR-Analyse wurden noch etwa 0.04 mg Tetraglyme pro g Produkt (I) nachgewiesen. Die Ausbeute betrug 183 g, d.h. 98.9 %.

Beispiel 6

8 kg des mit CsF, Tetraglyme und F113 verunreinigten HFPO-Roholigomers mit einem mittleren Molekulargewicht von 5500 g/mol wurde in einem 10 l-Rührkolben mit Ablaßhahn gegeben. Zum Roholigomer wurde eine Lösung von 28 g Chlortrimethylsilan in 2000 ml n-Hexan gegeben und bei Raumtemperatur 90 Minuten gerührt. Nach Phasentrennung wurde die untere Phase abgelassen, filtriert und im Vakuum bei 120°C von n-Hexan und F113 befreit. Das so gereinigte Produkt (I) enthielt gemäß NMR-Analyse noch etwa 0.09 mg Tetraglyme pro g Produkt (I). Nach Filtration und Destillation der oberen Phase (n-Hexan) wurden 45 g (91 %) des bei der Oligomerisation eingesetzten Tetraglymes und 35 g CsCl (inklusive der aus der unteren Phase abfiltrierten Menge) isoliert. Die Ausbeute betrug 7350 g, d.h. 99.3 %.

**Patentansprüche**

1. Verfahren zur Herstellung von Perfluorpolyetheracylfluoriden der Formel (I)

   $F_3C(CF)_2$-O-[$CF(CF_3)CF_2$-O-]$_n$$CF(CF_3)$-COF　(I)

   wobei n eine ganze Zahl von 0 - 60 bedeutet, dadurch gekennzeichnet, daß man Hexafluorpropylenoxid in Gegenwart von CsF und einem Polyethylenglykoldimethylether oligomerisiert und das dabei entstehende Reaktionsgemisch mit mindestens einer der in einem aprotischen Lösungsmittel gelösten Halogenverbindungen HCl, HBr, $BCl_3$, $PCl_3$, $SCl_2$ und $R_mSiCl_{4-m}$, wobei m = 0, 1, 2 oder 3 und R = $C_1$-$C_3$-Alkyl oder Phenyl ist, umsetzt.

2. Verfahren nach Anspruch 1, dadurch gekennzeichnet, daß man als Halogenverbindung Chlortrimethylsilan oder Chlorwasserstoff verwendet.

3. Verfahren nach Anspruch 1 oder 2, dadurch gekennzeichnet, daß man als Lösungsmittel n-Hexan oder Cyclohexan verwendet.